# DEMANDE DE BREVET EUROPEEN

(11) **EP 4 834 294 A1**
(43) Date de publication de la demande: **04.11.2026**
(21) Numéro de dépôt: 26161462.2
(22) Date de dépôt: 27.02.2026
(51) Int. Cl.: A61F 5/01

(54) **ORTHESE DE POIGNET**

(30) Priorité: 29.04.2025 FR 2504500
(71) Demandeur: LABORATOIRES INNOTHERA, 94110 Arcueil (FR)
(72) Inventeur: DUPORT, Guillaume, 42000 SAINT-ETIENNE (FR)
(74) Mandataire: Ipsilon

(57) **Abrégé**

L'orthèse (100) comprend une nappe (1) présentant un bord distal (2), un bord proximal (3), deux bords longitudinaux (4) et un orifice (5) de passage du pouce, la nappe étant enroulée sur elle-même avec ses bords longitudinaux adjacents, pour former une enveloppe (10) recevant la main et le poignet d'un utilisateur.

La nappe comprend :
- une bande centrale (11) formant une portion antérieure de l'enveloppe ;
- deux bandes d'extrémité (12) adjacentes chacune à l'un des bords longitudinaux de la nappe et formant ensemble une portion dorsale de l'enveloppe ;
- deux bandes de jonction (13) chacune reliant la bande centrale à l'une des bandes d'extrémité et formant une portion latérale de l'enveloppe.

Le bord proximal de la nappe est non rectiligne et la portion antérieure de l'enveloppe est plus longue que la portion dorsale et les portions latérales.

## Description

L'invention concerne une orthèse de poignet.

Plus particulièrement, l'invention concerne une telle orthèse qui comporte une nappe enroulée sur elle-même pour former une enveloppe recevant la main et le poignet d'un utilisateur. La nappe peut typiquement être maintenue dans un état serré au moyen d'une ou de plusieurs sangles enroulées autour d'elle.

Un utilisateur devant porter ce type d'orthèse peut choisir ou se voir prescrire :
- soit une orthèse longue, c'est-à-dire dont la partie couvrant l'avant-bras s'étend longitudinalement sur une assez grande distance, généralement d'au moins une fois voire une fois et demi la dimension de la paume dans la direction longitudinale, par exemple des métacarpes jusqu'aux deux tiers de l'avant bras ;
- soit une orthèse courte, c'est-à-dire dont la partie couvrant l'avant-bras s'étend longitudinalement sur une distance relativement limitée, généralement inférieure à la dimension de la paume dans la direction longitudinale, par exemple comprise entre 60% et 90% de ladite dimension de la paume. Une orthèse courte s'étend par exemple des métacarpes jusqu'au tiers de l'avant bras.

Une orthèse longue fournit une immobilisation très efficace du poignet, mais présente l'inconvénient d'être encombrante et peu respirante, ce qui a un impact très négatif sur l'observance. A l'inverse, une orthèse courte sera plus facilement et régulièrement portée, mais sa capacité d'immobilisation est moindre, la rendant moins efficace.

La présente invention vise à remédier aux inconvénients mentionnés ci-dessus.

A cet effet, l'invention concerne une orthèse de poignet comprenant une nappe présentant un bord distal, un bord proximal, deux bords longitudinaux et au moins un orifice de passage du pouce, la nappe étant enroulée sur elle-même de sorte que ses bords longitudinaux soient adjacents, pour former une enveloppe configurée pour recevoir la main et le poignet d'un utilisateur. La nappe présente une extrémité distale et comprend :
- une bande centrale qui forme sensiblement une portion antérieure de l'enveloppe, et dont le bord proximal forme une portion centrale du bord proximal de la nappe ;
- deux bandes d'extrémité qui sont chacune adjacente à l'un des bords longitudinaux de la nappe et dont les bords proximaux forment des portions d'extrémité du bord proximal de la nappe, lesdites deux bandes d'extrémité formant ensemble sensiblement une portion dorsale de l'enveloppe ;
- deux bandes de jonction reliant chacune la bande centrale à l'une des bandes d'extrémité, les bords proximaux des bandes de jonction formant chacun une portion de jonction du bord proximal de la nappe, chacune desdites deux bandes de jonction formant sensiblement une portion latérale de l'enveloppe.

Selon une définition générale de l'invention, chacune des portions de jonction du bord proximal de la nappe s'étend globalement en oblique entre la portion centrale et la portion d'extrémité correspondante du bord proximal de la nappe, et la distance longitudinale entre l'extrémité distale de la nappe et chacune des portions d'extrémité du bord proximal de la nappe est inférieure à la distance longitudinale entre l'extrémité distale de la nappe et la portion centrale du bord proximal de la nappe, de sorte que le bord proximal de la nappe est non rectiligne et que les bords proximaux de la portion dorsale et des portions latérales de l'enveloppe sont décalés dans la direction distale par rapport au bord proximal de la portion antérieure de l'enveloppe.

Ainsi, l'orthèse de poignet selon l'invention combine les avantages d'une orthèse longue et d'une orthèse courte, en minimisant les inconvénients, et sans réduire la performance de l'immobilisation.

Plus spécifiquement, l'enveloppe de l'orthèse selon l'invention présente une portion antérieure de longueur suffisante pour assurer une immobilisation efficace du poignet, en flexion / extension. En effet, cette immobilisation est en grande partie obtenue par l'appui fourni en face antérieure de la main et de l'avant-bras, cet appui étant d'autant plus performant qu'il est présent sur une grande distance longitudinale.

De plus, l'invention prévoit des portions latérales et dorsale de l'enveloppe plus courtes que la portion antérieure. Ceci n'empêche pas d'obtenir une immobilisation satisfaisante du poignet, dans la mesure où les portions latérales et dorsale de l'enveloppe participent à l'immobilisation du poignet sans assurer majoritairement cette fonction. En revanche, cela offre des avantages importants en termes de respirabilité, donc de confort de port, mais également de ressenti visuel allégé. L'utilisateur est ainsi moins réticent à porter régulièrement l'orthèse, ce qui favorise sa guérison ou l'atténuation des symptômes.

Selon un mode de réalisation, chacune des portions de jonction du bord proximal de la nappe forme une échancrure courbe. En d'autres termes, au voisinage de chacune de ces portions de jonction, la nappe présente une forme concave. En variante, chacune de ces portions de jonction pourrait être rectiligne.

Selon une réalisation possible, le bord proximal de la portion dorsale de l'enveloppe est décalé par rapport au bord proximal de la portion antérieure de l'enveloppe, dans la direction distale, d'une distance comprise entre 4 et 6 cm, par exemple d'environ 5 cm, ou comprise entre 15 et 30 % de la longueur de l'enveloppe, par exemple comprise entre 20 et 25%.

La largeur de la bande centrale peut être comprise entre 25 et 40% de la largeur de la nappe, ou entre 30 et 35%. La largeur de la bande centrale est par exemple d'environ 33% de la largeur de la nappe. La largeur de chaque bande d'extrémité peut être comprise entre 12 et 25% de la largeur de la nappe, ou entre 15 et 20%. La largeur de chaque bande d'extrémité est par exemple d'environ 16% de la largeur de la nappe. La largeur de chaque bande de jonction peut être comprise entre 12 et 25% de la largeur de la nappe, ou entre 15 et 20%. La largeur de chaque bande de jonction est par exemple d'environ 18% de la largeur de la nappe. Le terme « largeur » correspond à la plus grande distance orthogonalement à l'axe longitudinal.

L'orthèse peut comprendre un organe de rigidification dorsal agencé au voisinage de chacun des bords longitudinaux de la nappe, ledit organe de rigidification dorsal comportant une portion centrale qui s'étend dans une bande d'extrémité sensiblement longitudinalement :
- sur au moins 30% de la longueur de l'enveloppe, ou au moins 35%, par exemple environ 40% de la longueur de l'enveloppe ;
- ou sur au moins 45%, ou au moins 50%, par exemple environ 55% de la dimension longitudinale totale de l'organe de rigidification dorsal.

En outre, chaque organe de rigidification dorsal peut comporter une portion distale qui s'étend depuis l'extrémité distale de la portion centrale en étant inclinée dans la direction distale et à l'opposé du bord longitudinal voisin (c'est-à-dire le bord longitudinal au voisinage duquel l'organe de rigidification dorsal en question est situé), par rapport à la direction longitudinale, d'un angle compris entre 130 et 160°, par exemple d'environ 145°. Ladite portion distale a une dimension longitudinale comprise entre 15 et 30%, ou entre 20 et 25%, de la dimension longitudinale totale de l'organe de rigidification dorsal. Ladite portion distale s'étend au moins partiellement dans la bande de jonction adjacente, lesdites portions distales étant configurées pour être placées de part et d'autre de la main, en position d'utilisation, sensiblement en regard des métacarpiens de l'index et de l'auriculaire.

Ainsi, une fois l'orthèse selon l'invention en place sur la main et l'avant-bras de l'utilisateur, les portions distales des organes de rigidification dorsaux viennent encadrer la main et jouent en partie de la fonction d'immobilisation en empêchant les mouvements d'abduction et d'adduction du poignet. Par conséquent, la sangle distale traditionnellement utilisée s'avère superflue, un serrage plus léger et discret étant suffisant, par exemple via un lacet. En outre, du fait de l'efficacité du maintien procuré par les portions distales des organes de rigidification dorsaux, il n'est pas nécessaire de prévoir une enveloppe épaisse ni s'étendant assez loin dans la direction distale. Il s'ensuit que l'orthèse selon l'invention présente des avantages importants en termes de respirabilité, donc de confort de port, mais également de ressenti visuel allégé.

De plus, chaque organe de rigidification dorsal peut comporter une portion proximale qui s'étend depuis l'extrémité proximale de la portion centrale en étant inclinée dans la direction proximale et à l'opposé du bord longitudinal voisin (c'est-à-dire le bord longitudinal au voisinage duquel l'organe de rigidification dorsal en question est situé), par rapport à la direction longitudinale, d'un angle compris entre 145 et 175°, par exemple voisin de 160°. Ladite portion proximale a une dimension longitudinale comprise entre 15 et 30%, ou entre 20 et 25%, de la dimension longitudinale totale de l'organe de rigidification dorsal.

Selon une réalisation possible, l'orthèse comprend un organe de rigidification antérieur qui comporte une portion distale bombée dans la direction postérieure, destinée à être reçue dans la paume de la main, prolongée par une plaque allongée s'étendant sensiblement jusqu'au bord proximal de la nappe. Cet organe de rigidification antérieur contribue à assurer l'immobilisation du poignet, par un effet de bras de levier important.

L'orthèse peut comprendre un dispositif de serrage comportant :
- une pluralité d'œillets agencés dans la nappe le long de chacun des bords longitudinaux ;
- une patte de traction comportant deux œillets et possédant des moyens d'accrochage amovible sur l'enveloppe ;
- et un lacet formant une boucle fermée, le lacet possédant une partie de laçage passant successivement dans les œillets de la nappe, et une partie de serrage comportant un premier brin et un deuxième brin s'étendant vers l'extérieur de l'enveloppe respectivement depuis un premier œillet et un deuxième œillet de la nappe, les parties extrêmes des deux brins passant chacune dans un œillet de la patte de traction et étant reliées par un brin de liaison ;
le dispositif de serrage étant configuré de sorte qu'une traction exercée sur la patte de traction, sensiblement orthogonalement à direction longitudinale, conduise au rapprochement des bords longitudinaux de l'enveloppe grâce au lacet, le dispositif de serrage étant alors à l'état serré, le lacet possédant une longueur suffisante pour que, à l'état serré, la partie de serrage puisse être enroulée autour de l'enveloppe sur au moins un tour, l'accrochage amovible de la patte de traction sur l'enveloppe permettant de maintenir le dispositif de serrage à l'état serré.

Par exemple, le premier œillet est situé au voisinage de l'un des bords longitudinaux de l'enveloppe et du bord proximal de l'enveloppe, et le deuxième œillet est situé au voisinage du même bord longitudinal et de préférence adjacent au premier œillet, dans la direction distale.

Les bords longitudinaux de la nappe peuvent être incurvés, lorsque la nappe est dans un état plan.

Selon une réalisation possible, lorsque la nappe est dans un état plan, elle présente une ligne longitudinale médiane de symétrie, la nappe présentant deux orifices de passage du pouce, de sorte que l'orthèse est ambidextre.

L'orthèse peut en outre comprendre un pan de textile assemblé entre les bandes d'extrémité de la nappe, lorsque la nappe est enroulée sur elle-même, de sorte que l'orthèse forme un manchon circonférentiellement fermé dans lequel un utilisateur peut insérer sa main sensiblement longitudinalement.

On décrit à présent, à titre d'exemple non limitatif, un mode de réalisation possible de l'invention, en référence aux figures annexées :
[FIG 1] est une vue en plan d'une nappe appartenant à une orthèse de poignet selon un mode de réalisation de l'invention ;
[FIG 2] est une vue dorsale d'une orthèse comportant la nappe de la figure 1 enroulée sur elle-même ;
[FIG 3] est une vue antérieure d'un organe de rigidification antérieur de l'orthèse ;
[FIG 4] est une vue latérale de l'organe de rigidification antérieur de la figure 3 ;
[FIG 5] illustre une variante de l'orthèse de la figure 2 ;
[FIG 6], [FIG 7] et [FIG 8] représentent l'orthèse mise en place sur le poignet d'un utilisateur, respectivement en vue antérieure, en vue dorsale et en vue latérale.

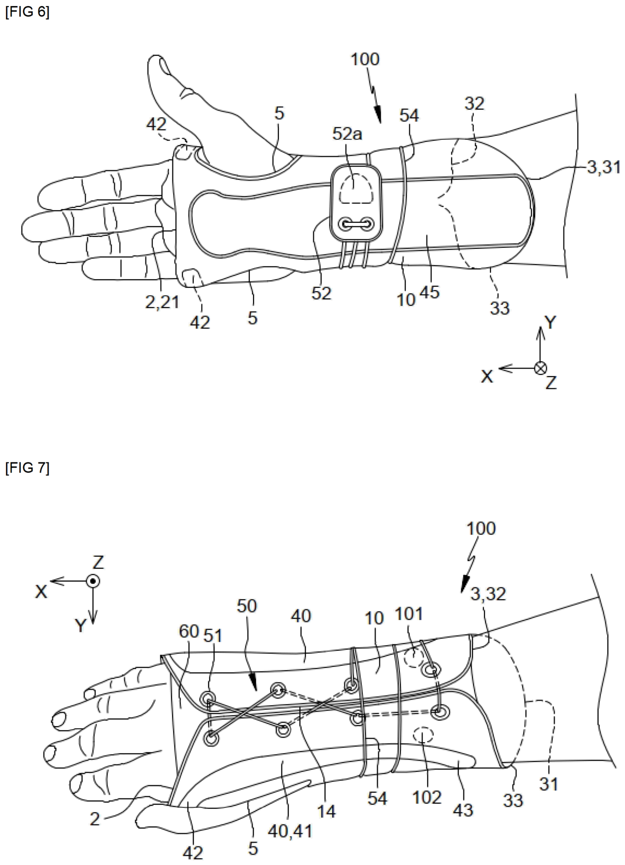

L'invention concerne une orthèse 100 de poignet destinée à être placée autour du poignet d'un utilisateur ainsi que, partiellement, autour de sa main, de son pouce et de son avant-bras, comme on le voit sur les figures 6 à 8.

On définit la direction longitudinale X comme la direction générale de l'avant-bras. Par rapport à cette direction X, on emploie le terme « distal » pour désigner un élément situé plus près de l'extrémité des doigts, par opposition au terme « proximal » qui est employé pour désigner un élément situé plus près du coude.

On définit également la direction transversale Y qui est orthogonale à la direction longitudinale X et correspond à l'axe latéral / médial de l'avant-bras. Le terme « latéral » est utilisé en référence à la direction Y.

Enfin, on définit la direction Z orthogonale aux directions X et Y, et qui correspond à l'axe antéro-postérieur de l'avant-bras. Par rapport à cette direction Z, on emploie le terme « antérieur » pour désigner un élément situé du côté de la paume de la main, par opposition au terme « dorsal ».

L'orthèse 100 comprend une nappe 1.

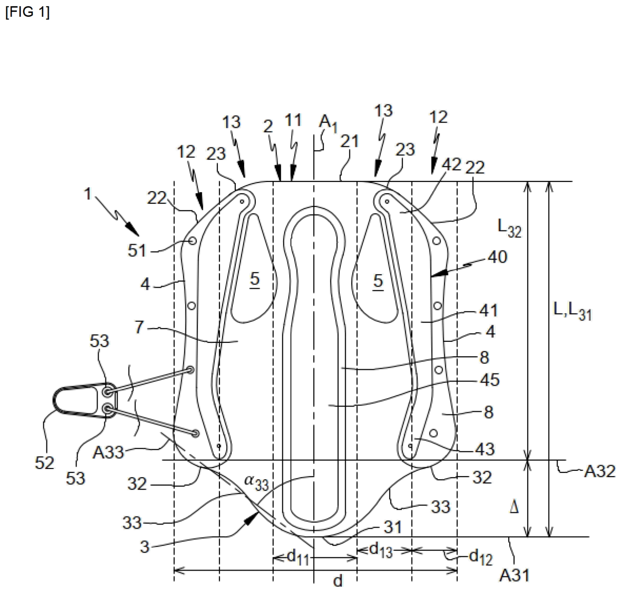

La nappe 1 peut être initialement sensiblement plane, comme illustré sur la figure 1. La nappe 1 présente une ligne médiane A1 qui, en position portée de l'orthèse, est orientée sensiblement longitudinalement.

La nappe 1 possède un bord distal 2, un bord proximal 3 et deux bords longitudinaux 4, ainsi qu'au moins un orifice 5 de passage du pouce.

Selon une réalisation possible mais optionnelle, l'orthèse 100 est conçue pour être ambidextre. Dans ce cas, la nappe 1 possède deux orifices 5 de passage du pouce, et la ligne médiane A1 est une ligne de symétrie de la nappe 1.

Dans l'orthèse 100, la nappe 1 est enroulée sur elle-même de sorte que ses bords longitudinaux 4 soient adjacents, pour former une enveloppe 10 configurée pour recevoir une partie de la main, le poignet, et une partie de l'avant-bras de l'utilisateur. La nappe 1 présente une face intérieure 6, tournée vers le poignet en position portée de l'orthèse 100, et une face extérieure 7. La nappe 1 est donc la version ouverte et à plat de l'enveloppe 10 qui, elle, est enroulée.

La nappe 1 peut comporter plusieurs trous d'aération (non représentés).

La surface de la nappe 1 peut être décomposée en un certain nombre de bandes, chaque bande s'étendant de préférence du bord distal 2 au bord proximal 3 de la nappe 1 et étant de préférence délimitée par des lignes longitudinales.

Ainsi, la nappe 1 comprend une bande centrale 11 qui forme sensiblement une portion antérieure de l'enveloppe 10, c'est-à-dire qui est située contre la face antérieure de la main et du poignet en position portée de l'orthèse 100. La bande centrale 11 présente un bord distal qui forme la portion centrale 21 du bord distal 2 de la nappe 1, et un bord proximal qui forme la portion centrale 31 du bord proximal 3 de la nappe 1.

La nappe 1 comprend également deux bandes d'extrémité 12. Chaque bande d'extrémité 12 est adjacente à l'un des bords longitudinaux 4 de la nappe 1 et présente d'une part un bord distal qui forme une portion d'extrémité 22 du bord distal 2 de la nappe 1, et d'autre part un bord proximal qui forme une portion d'extrémité 32 du bord proximal 3 de la nappe 1. Les deux bandes d'extrémité 12 forment ensemble sensiblement une portion dorsale de l'enveloppe 10, c'est-à-dire qui est située contre la face dorsale de la main et du poignet en position portée de l'orthèse 100.

De plus, la nappe 1 comprend deux bandes de jonction 13. Chaque bande de jonction 13 relie la bande centrale 11 à l'une des bandes d'extrémité 12. Chaque bande de jonction 13 présente d'une part un bord distal qui forme une portion de jonction 23 du bord distal 2 de la nappe 1, et d'autre part un bord proximal qui forme une portion de jonction 33 du bord proximal 3 de la nappe 1. Chacune des deux bandes de jonction 13 forme sensiblement une portion latérale de l'enveloppe 10, c'est-à-dire une portion qui est située contre l'une des faces latérales de la main et du poignet en position portée de l'orthèse 100.

De préférence, les bandes 11, 12, 13 de la nappe 1 sont jointives selon la direction transversale Y, c'est-à-dire que la nappe 1 est formée des cinq bandes précédemment décrites et dépourvue de bandes intermédiaires. Avec cette réalisation, les portions précitées du bord distal 2, respectivement du bord proximal 3, de la nappe 1 sont également jointives.

Par exemple, la largeur d11 de la bande centrale 11 est comprise entre 25 et 40% de la largeur d1 de la nappe 1, la largeur d12 de chaque bande d'extrémité 12 est comprise entre 12 et 25% de la largeur d1 de la nappe 1, et/ou la largeur d13 de chaque bande de jonction 13 est comprise entre 12 et 25% de la largeur d1 de la nappe 1. La largeur est la dimension orthogonalement à la ligne médiane A1.

Le bord distal 2 de la nappe 1 est aussi le bord distal de l'enveloppe 10, ce bord étant de préférence plan lorsqu'il s'agit de la nappe 1 mais enroulé lorsqu'il s'agit de l'enveloppe 10. De façon similaire, le bord proximal 3 de la nappe 1, qui est de préférence plan, est aussi le bord proximal de l'enveloppe 10, qui est enroulé.

La nappe 1 présente une extrémité distale qui est la partie la plus distale du bord distal 2.

Selon une réalisation possible, le bord distal 2 de la nappe 1 peut présenter les caractéristiques suivantes :
- la portion centrale 21 s'étend sensiblement transversalement, c'est-à-dire orthogonalement à la ligne médiane A1, ou présente une ligne moyenne sensiblement transversale ;
- chacune des portions d'extrémité 22 s'étend en oblique depuis l'un des bords longitudinaux 4 de la nappe 1 jusqu'à la portion de jonction 23 correspondante, en étant incliné dans la direction distale, chacune des portions d'extrémité 22 étant par exemple sensiblement rectiligne ;
- et chacune des portions de jonction 23 s'étend dans le prolongement de la portion centrale 21 puis se courbe pour rejoindre la portion d'extrémité 22 correspondante.

Avec cette configuration, l'extrémité distale de la nappe 1 est située sur la portion centrale 21 du bord distal 2.

La portion centrale 31 du bord proximal 3 de la nappe 1 peut s'étendre sensiblement transversalement, c'est-à-dire orthogonalement à la ligne médiane A1, ou présenter une ligne moyenne A31 sensiblement transversale, comme illustré sur la figure 1.

On peut également définir pour chacune des portions d'extrémité 32 du bord proximal 3 une ligne moyenne A32 sensiblement transversale. Chacune de ces portions d'extrémité 32 peut être courbe.

Chacune des portions de jonction 33 du bord proximal 3 de la nappe 1 relie la portion centrale 31 à l'une des portions d'extrémité 32 dudit bord proximal 3. De préférence, chacune des portions de jonction 33 est située longitudinalement entre la portion centrale 31 et les portions d'extrémité 32. En d'autres termes, chacune des portions de jonction 33 ne s'étend pas au-delà de la portion centrale 31, dans la direction proximale.

Selon une réalisation possible, chacune des portions de jonction 33 du bord proximal 3 de la nappe 1 s'étend globalement en oblique entre la portion centrale 31 et la portion d'extrémité 32 correspondante du bord proximal 3 de la nappe 1. En d'autres termes, chacune des portions de jonction 33 présente une ligne moyenne A33 qui forme un angle α33 non nul et non droit avec la ligne médiane A1 de la nappe 1, comme illustré sur la figure 1. Par exemple, l'angle α33 peut être compris entre 45 et 60°.

De plus, on peut prévoir que la distance longitudinale L32 entre l'extrémité distale de la nappe 1 et chacune des portions d'extrémité 32 du bord proximal 3 de la nappe 1 soit inférieure à la distance longitudinale L31 entre l'extrémité distale de la nappe 1 et la portion centrale 31 du bord proximal 3 de la nappe 1. La distance longitudinale est considérée parallèlement à la ligne médiane A1, jusqu'à la ligne transversale moyenne de la portion considérée du bord proximal 3. Par exemple, la distance longitudinale L32 peut être voisine de 170 mm et la distance longitudinale L31 peut être voisine de 230 mm.

En conséquence, le bord proximal 3 de la nappe 1 est non rectiligne. En d'autres termes, le bord proximal de l'enveloppe 10 n'est pas situé dans un plan transversal de l'avant-bras, à savoir un plan orthogonal à la direction longitudinale X. De plus, les bords proximaux de la portion dorsale de l'enveloppe 10 (c'est-à-dire les portions d'extrémité 32 du bord proximal 3) et les bords proximaux des portions latérales de l'enveloppe 10 (c'est-à-dire les portions de jonction 33 du bord proximal 3) sont décalés dans la direction distale par rapport au bord proximal de la portion antérieure de l'enveloppe 10 (c'est-à-dire la portion centrale 31 du bord proximal 3). En d'autres termes, l'enveloppe 10 est plus courte dans sa portion dorsale et ses portions latérales que dans sa portion antérieure, comme on le voit bien sur les figures 6 à 8.

Par exemple, le bord proximal de la portion dorsale de l'enveloppe 10 peut être décalé par rapport au bord proximal de la portion antérieure de l'enveloppe 10, dans la direction distale, d'une distance Δ comprise entre 4 et 6 cm, ou comprise entre 15 et 30 % de la longueur L de l'enveloppe 10. La longueur L de l'enveloppe 10, qui est aussi la longueur L de la nappe 1, est la plus grande distance de la nappe 1 le long de la ligne médiane A1, c'est-à-dire la distance entre l'extrémité distale et l'extrémité proximale de la nappe 1. La longueur L peut être égale à la distance L31.

Dans la réalisation illustrée à titre d'exemple, qui est non limitative, chacune des portions de jonction 33 du bord proximal 3 de la nappe 1 forme une échancrure courbe, dont la concavité est de préférence dirigée vers l'extérieur.

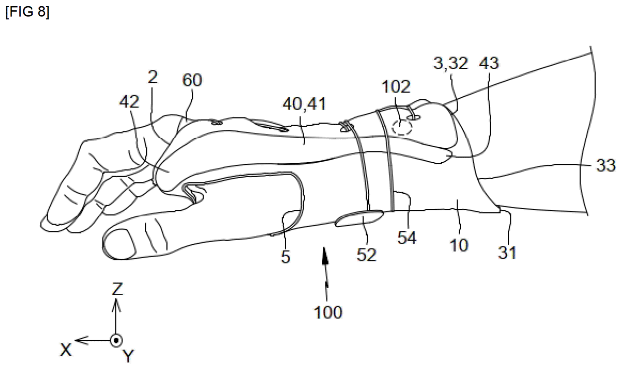

Il est à noter que, comme cela apparaît notamment sur la figure 8, chacune des portions de jonction 33 du bord proximal 3 de l'enveloppe 10 est en regard de l'une des faces latérales de l'avant-bras et s'étend sur une grande partie de ladite face latérale. Une portion de jonction 33 n'est pas réduite à un simple congé de raccordement localisé, par exemple au voisinage d'un bord longitudinal 4.

Selon un mode de réalisation, les bords longitudinaux 4 de la nappe 1 peuvent être incurvés, lorsque la nappe 1 est dans un état plan, comme on le voit sur la figure 1.

Selon une réalisation possible, l'orthèse 100 peut par ailleurs comprendre un ou plusieurs organes de rigidification. Ceux-ci sont de préférence assemblés contre la face extérieure 7 de la nappe 1. A cet effet, il peut être prévu localement un textile 8 superposé à la nappe 1 et soudé à celle-ci, de façon que l'organe de rigidification soit emprisonné entre ce textile 8 et la face extérieure 7 de la nappe 1. Le textile 8 peut être un tissu velours.

La face intérieure 6 de la nappe peut être dépourvue de tout élément rapporté et être lisse.

L'orthèse 100 peut comprendre un organe de rigidification dorsal 40 agencé au voisinage de chacun des bords longitudinaux 4 de la nappe 1.

Chaque organe de rigidification dorsal 40 peut comporter une portion centrale 41 qui s'étend dans la bande d'extrémité 12 correspondante, sensiblement longitudinalement c'est-à-dire sensiblement parallèlement à la ligne médiane A1. Par exemple, ladite portion centrale 41 s'étend sur au moins 30% de la longueur L de l'enveloppe 10 ou sur au moins 45% de la dimension longitudinale totale de l'organe de rigidification dorsal 40. En outre, de préférence, ladite portion centrale 41 s'étend sur moins de 65% ou moins de 60% de la dimension longitudinale totale de l'organe de rigidification dorsal 40.

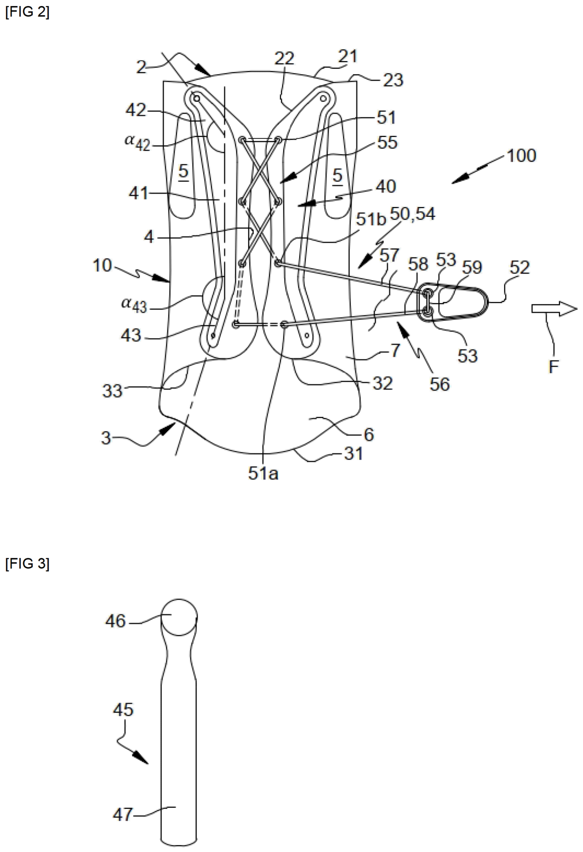

Chaque organe de rigidification dorsal 40 peut aussi comporter une portion distale 42 qui s'étend depuis l'extrémité distale de la portion centrale 41, en étant inclinée dans la direction distale et à l'opposé du bord longitudinal 4 dont elle est voisine, c'est-à-dire en direction de la ligne médiane A1 sur une nappe 1 sensiblement plane. Par exemple, ladite portion distale 42 est inclinée par rapport à la direction longitudinale X d'un angle α42 compris entre 130 et 160°, comme illustré sur la figure 2. Ladite portion distale 42 présente une dimension longitudinale comprise entre 15 et 30% de la dimension longitudinale totale de l'organe de rigidification dorsal 40. Elle s'étend au moins partiellement dans la bande de jonction 13 adjacente à la bande d'extrémité 12 dans laquelle est située la portion centrale 41 de l'organe de rigidification dorsal 40. Par exemple, la portion distale 42 s'étend sensiblement jusqu'au bord distal 2 de la nappe 1, dans la bande de jonction 12.

Chaque organe de rigidification dorsal 40 peut aussi comporter une portion proximale 43 qui s'étend depuis l'extrémité proximale de la portion centrale 41, en étant inclinée dans la direction proximale et à l'opposé du bord longitudinal 4 dont elle est voisine. Par exemple, ladite portion proximale 43 est inclinée par rapport à la direction longitudinale X d'un angle α43 compris entre 145 et 175°. Ladite portion proximale 43 présente une dimension longitudinale comprise entre 15 et 30% de la dimension longitudinale totale de l'organe de rigidification dorsal 40. Par exemple, la portion proximale 43 s'étend sensiblement jusqu'au bord proximal 3 de la nappe 1, dans la bande d'extrémité 13 ou la bande de jonction 12.

On peut prévoir que l'organe de rigidification dorsal 40 s'étende sensiblement du bord distal 2 jusqu'au bord proximal 3, dans les bandes de la nappe 1 dans lesquelles il est situé, à savoir une bande d'extrémité 12 et la bande de jonction 13 adjacente.

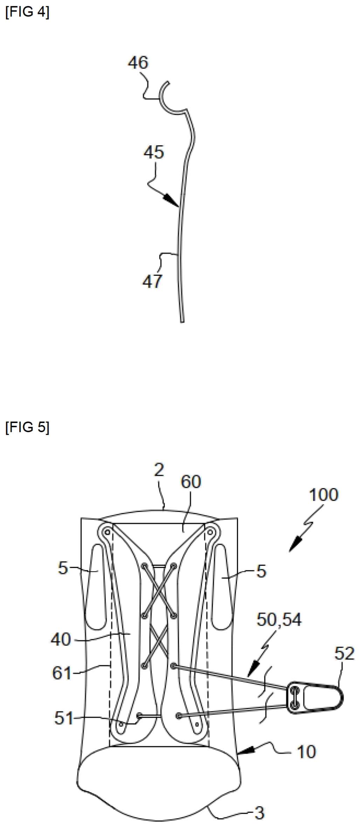

L'orthèse 100 peut également comprendre un organe de rigidification antérieur 45. Selon un mode de réalisation, et comme illustré sur les figures 3 et 4, l'organe de rigidification antérieur 45 peut comporter une portion distale 46 présentant une forme bombée dans la direction postérieure, destinée à être reçue dans la paume de la main, prolongée par une plaque 47 allongée s'étendant sensiblement jusqu'au bord proximal 3 de la nappe 1.

La portion distale 46 peut présenter sensiblement la forme d'une demi-sphère creuse. La plaque 47 peut avoir une forme suivant la morphologie de la face antérieure de la main et de l'avant-bras quand le poignet est en position neutre, en particulier quand il n'est ni en flexion ni en extension.

L'orthèse peut également comprendre un dispositif de serrage 50, permettant éventuellement de maintenir la nappe 1 enroulée sous la forme de l'enveloppe 10, et le serrage approprié de l'enveloppe 10 autour de la main, du poignet et de l'avant-bras de l'utilisateur.

Le dispositif de serrage 50 comporte :
- une pluralité d'œillets 51 agencés dans la nappe 1 le long de chacun des bords longitudinaux 4 ;
- une patte de traction 52 comportant deux œillets 53 et possédant des moyens d'accrochage amovible sur l'enveloppe 10, par exemple une zone de crochets 52a d'un système de type Velcro^{®} ;
- et un lacet 54 formant une boucle fermée. Le lacet 54 possède une partie de laçage 55 passant successivement dans les œillets 51 de la nappe 1, et une partie de serrage 56 comportant un premier brin 57 et un deuxième brin 58 s'étendant vers l'extérieur de l'enveloppe 10 respectivement depuis un premier œillet 51a et un deuxième œillet 51b de la nappe 1, les parties extrêmes des deux brins 57, 58 passant chacune dans un œillet 53 de la patte de traction 52 et étant reliées par un brin de liaison 59.

De préférence, le premier œillet 51a et le deuxième œillet 51b sont situés le long d'un même bord longitudinal 4 et/ou sont décalés l'un par rapport à l'autre selon la direction longitudinale X. Par exemple, le premier œillet 51a peut être situé au voisinage de l'un des bords longitudinaux 4 de l'enveloppe 10 et du bord proximal 3 de l'enveloppe 10, tandis que le deuxième œillet 51b peut être situé au voisinage du même bord longitudinal 4 et de préférence adjacent au premier œillet 51a, dans la direction distale.

Comme on le voit sur la figure 5, l'orthèse 100 peut en outre comprendre un pan de textile 60 assemblé entre les bandes d'extrémité 12 de la nappe 1, lorsque la nappe 1 est enroulée sur elle-même, de sorte que l'orthèse 100 forme un manchon circonférentiellement fermé dans lequel un utilisateur peut insérer sa main sensiblement longitudinalement. La présence de ce pan de textile 60 rend l'orthèse 100 plus facile à enfiler et, de plus, permet de protéger la peau du porteur du lacet 54, en particulier lors du serrage du lacet 54. Le pan de textile 60 peut se présenter sous la forme d'un tissu fin qui est assemblé à la face intérieure 6 de la nappe 1, le long de ses bords longitudinaux 61, par exemple par couture. Le pan de textile 60 peut avantageusement être élastique.

Pour la mise en place de l'orthèse 100, l'utilisateur insère sa main dans l'enveloppe 10, place son pouce dans l'orifice 5 approprié et tire l'enveloppe 10 dans la direction proximale jusqu'à ce que le pouce soit correctement positionné dans l'orifice 5.

L'utilisateur procède ensuite au serrage de l'orthèse 100 au moyen du dispositif de serrage 50. A cet effet, l'utilisateur exerce une traction sur la patte de traction 52, par exemple sensiblement orthogonalement à direction longitudinale, comme illustré par la flèche F sur la figure 2. Cette traction conduit au rapprochement des bords longitudinaux 4 de l'enveloppe 10 grâce au lacet 54, et le dispositif de serrage 50 est alors à l'état serré. De préférence, le lacet 54 possède une longueur suffisante pour que, à l'état serré, la partie de serrage 56 puisse être enroulée autour de l'enveloppe 10 sur au moins un tour. Enfin, la patte de traction 52 est attachée de façon amovible sur l'enveloppe 10, par exemple grâce à la zone de crochets 52a, ce qui permet de maintenir le dispositif de serrage 50 à l'état serré.

La forme incurvée des bords longitudinaux 4 de la nappe 1, lorsque celle-ci est à l'état plan, se traduit en position d'utilisation, c'est-à-dire sur la nappe 1 enroulée, par l'obtention d'une enveloppe 10 de forme gauche, vue latéralement, cette forme gauche épousant la forme conique de l'avant-bras comme on le voit sur la figure 8, en vue latérale. Les bords longitudinaux 4 sont alors adjacents, plus ou moins proches selon le degré de serrage. Les bords longitudinaux 4 sont par exemple sensiblement longitudinaux et parallèles, comme on le voit sur la figure 7.

En position portée de l'orthèse 100, les organes de rigidification dorsaux 40 se trouvent agencés comme exposé ci-après.

La portion centrale 41 de chaque organe de rigidification dorsal 40 est située contre la face dorsale du membre, incluant la main, le poignet et l'avant-bras, à proximité de l'une des faces latérales du membre.

Les portions distales 42 sont configurées pour être placées de part et d'autre de la main, sensiblement en regard des métacarpiens de l'index et de l'auriculaire. Plus précisément, par exemple, les parties extrêmes distales des portions distales 42 sont situées sensiblement en regard - transversalement - de la tête de l'os métacarpien de l'index, respectivement de l'auriculaire. En conséquence, les mouvements d'abduction et d'adduction du poignet sont considérablement limités voire empêchés. L'obtention d'une telle restriction de ces mouvements est d'autant plus importante dans le cas d'une orthèse 100 ambidextre, du fait de la présence d'un orifice 5 de passage de pouce additionnel et non utilisé, qui tend à rendre l'orthèse moins rigide et empêche de prévoir un organe de rigidification dans la zone au voisinage de cet orifice 5 additionnel.

Les portions proximales 43 s'écartent l'une de l'autre transversalement, dans la direction proximale. Ceci apporte un certain nombre d'avantages.

Cet agencement spécifique permet d'éviter que les organes de rigidification dorsaux 40 ne se trouvent en regard du processus styloïde de l'ulna 101 ou du tubercule dorsal du radius 102, ce qui serait source d'inconfort. Plus précisément, les organes de rigidification dorsaux 40 sont de préférence conçus pour que la zone de liaison entre la portion centrale 41 et la portion proximale 42 soit située du côté distal par rapport au processus styloïde de l'ulna 101 et au tubercule dorsal du radius 102.

En outre, selon un mode de réalisation, chaque portion proximale 43 s'étend dans une bande d'extrémité 12 de la nappe 1 mais également au moins partiellement dans la bande de jonction 13 adjacente. Les parties extrêmes proximales des portions proximales 43 sont alors situées de part et d'autre de l'avant-bras du porteur. Dans ce cas, les portions proximales 43 peuvent former des zones additionnelles d'appui latéral, comme cela apparaît notamment sur la figure 8, et ainsi améliorer la stabilisation latérale du poignet. En variante cependant, on pourrait prévoir que chaque portion proximale 43 soit intégralement située dans une bande d'extrémité 12 de la nappe 1.

De plus, en prévoyant un écartement mutuel des portions proximales 43 au lieu d'organes de rigidification dorsaux 40 longitudinaux, on évite que les organes de rigidification dorsaux 40 n'appuient trop fort sur la face dorsale du membre, du fait du diamètre croissant de l'avant-bras dans la direction proximale. A nouveau, ceci permet d'améliorer le confort de port.

Avec la configuration décrite, les organes de rigidification dorsaux 40 présentent une forme morphologique qui permet à la fois d'assurer une immobilisation performante et de garantir un confort de port satisfaisant.

Par ailleurs, puisqu'elle présente une portion postérieure et des portions latérales relativement courtes, l'orthèse 100 offre un aspect visuel plus léger et moins dissuasif. Cependant, l'orthèse 100 conserve une portion antérieure de longueur suffisante pour assurer une immobilisation efficace du poignet, en flexion / extension, en assurant un appui approprié. Cet appui peut être renforcé par la présence de l'organe de rigidification antérieur 45, notamment par la plaque 47 allongée qui peut former le contre-appui des organes de rigidification dorsaux 40. La forme bombée de la portion distale 46, en étant logée dans la paume, contraint la position de la main, ce qui contribue à la limitation des mouvements du poignet. L'efficacité de l'immobilisation est ainsi améliorée et, en conséquence, il est possible d'obtenir une orthèse 100 performante avec une nappe 1 plus fine.

Le dispositif de serrage 50 peut également participer à la restriction des mouvements du poignet sans dégrader le confort.

Le lacet 54 permet de plaquer les organes de rigidification contre le membre du porteur, et ainsi de renforcer le système d'appui et contre-appui, ce qui contribue à compenser le fait que la partie dorsale de l'orthèse 100 est plus courte. De plus, la partie de serrage 56, qui vient s'enrouler autour de l'enveloppe 10 de préférence dans la moitié proximale de l'enveloppe 10, réalise ce placage au niveau de la zone de flexion du poignet, et renforce donc l'immobilisation.

Cependant, cet avantage n'est pas obtenu au détriment du confort. En particulier, le positionnement particulier et l'espacement suffisant du premier œillet 51a et du deuxième œillet 51b de la nappe 1 permet d'éviter que le lacet 54 n'appuie sur le processus styloïde de l'ulna 101 ou sur le tubercule dorsal du radius 102, comme cela est visible sur les figures 7 et 8. Par exemple, les premier et deuxième œillets 51a, 51b sont espacés d'au moins 3 ou 4 ou 5 cm, et peuvent ainsi se placer de part et d'autre - selon la direction longitudinale X - du processus styloïde de l'ulna 101 et du tubercule dorsal du radius 102.

Dans la réalisation représentée, les œillets 51 de la nappe 1 sont agencés entre l'organe de rigidification dorsal 40 et le bord longitudinal 4 adjacent. En variante, tout ou partie des œillets 51, par exemple les œillets centraux, pourraient être disposés à proximité de l'organe de rigidification dorsal 40, mais du côté de la ligne médiane A1. Un tel agencement permet de faire plus facilement se chevaucher les bandes d'extrémité 12, pour une meilleure adaptation de l'orthèse à la morphologie du porteur, dans une plus grande gamme de tailles. De plus, cela permet un appui renforcé sur les organes de rigidification dorsaux 40, de préférence au niveau de la zone de flexion du poignet, donc une amélioration de l'immobilisation.

Il va de soi que l'invention n'est pas limitée au mode de réalisation décrit ci-dessus à titre d'exemple mais qu'elle comprend tous les équivalents techniques et les variantes des moyens décrits ainsi que leurs combinaisons.

## Revendications

1. Orthèse (100) de poignet, comprenant une nappe (1) présentant un bord distal (2), un bord proximal (3), deux bords longitudinaux (4) et au moins un orifice (5) de passage du pouce, la nappe (1) étant enroulée sur elle-même de sorte que ses bords longitudinaux (4) soient adjacents, pour former une enveloppe (10) configurée pour recevoir la main et le poignet d'un utilisateur, la nappe (1) présentant une extrémité distale et comprenant :
- une bande centrale (11) qui forme sensiblement une portion antérieure de l'enveloppe (10), et dont le bord proximal forme une portion centrale (31) du bord proximal (3) de la nappe (1) ;
- deux bandes d'extrémité (12) qui sont chacune adjacente à l'un des bords longitudinaux (4) de la nappe (1) et dont les bords proximaux forment des portions d'extrémité (32) du bord proximal (3) de la nappe (1), lesdites deux bandes d'extrémité (12) formant ensemble sensiblement une portion dorsale de l'enveloppe (10) ;
- deux bandes de jonction (13) reliant chacune la bande centrale (11) à l'une des bandes d'extrémité (12), les bords proximaux des bandes de jonction (13) formant chacun une portion de jonction (32) du bord proximal (3) de la nappe (1), chacune desdites deux bandes de jonction (13) formant sensiblement une portion latérale de l'enveloppe (10) ;
**caractérisée en ce que** chacune des portions de jonction (32) du bord proximal (3) de la nappe (1) s'étend globalement en oblique entre la portion centrale (31) et la portion d'extrémité (32) correspondante du bord proximal (3) de la nappe (1) et **en ce que** la distance longitudinale (L32) entre l'extrémité distale de la nappe (1) et chacune des portions d'extrémité (32) du bord proximal (3) de la nappe (1) est inférieure à la distance longitudinale (L31) entre l'extrémité distale de la nappe (1) et la portion centrale (31) du bord proximal (3) de la nappe (1), de sorte que le bord proximal (3) de la nappe (1) est non rectiligne et que les bords proximaux de la portion dorsale et des portions latérales de l'enveloppe (10) sont décalés dans la direction distale par rapport au bord proximal de la portion antérieure de l'enveloppe (10).

2. Orthèse selon la revendication 1, **caractérisée en ce que** chacune des portions de jonction (33) du bord proximal (3) de la nappe (1) forme une échancrure courbe.

3. Orthèse selon la revendication 1 ou 2, **caractérisée en ce que** le bord proximal (3) de la portion dorsale de l'enveloppe (10) est décalé par rapport au bord proximal (3) de la portion antérieure de l'enveloppe (10), dans la direction distale, d'une distance (Δ) comprise entre 4 et 6 cm, ou comprise entre 15 et 30 % de la longueur (L) de l'enveloppe (10).

4. Orthèse selon l'une des revendications 1 à 3, **caractérisée en ce que** la largeur (d11) de la bande centrale (11) est comprise entre 25 et 40% de la largeur (d) de la nappe (1), et/ou **en ce que** la largeur (d12) de chaque bande d'extrémité (12) est comprise entre 12 et 25% de la largeur (d) de la nappe (1), et/ou **en ce que** la largeur (d13) de chaque bande de jonction (13) est comprise entre 12 et 25% de la largeur (d) de la nappe (1).

5. Orthèse selon l'une des revendications 1 à 4, **caractérisée en ce qu'**elle comprend un organe de rigidification dorsal (40) agencé au voisinage de chacun des bords longitudinaux (4) de la nappe (1), ledit organe de rigidification dorsal (40) comportant une portion centrale (41) qui s'étend dans une bande d'extrémité (12) sensiblement longitudinalement, sur au moins 30% de la longueur (L) de l'enveloppe (10) ou sur au moins 45% de la dimension longitudinale totale de l'organe de rigidification dorsal (40).

6. Orthèse selon la revendication 5, **caractérisée en ce que** chaque organe de rigidification dorsal (40) comporte une portion distale (42) qui s'étend depuis l'extrémité distale de la portion centrale (41) en étant inclinée dans la direction distale et à l'opposé du bord longitudinal (4) voisin, par rapport à la direction longitudinale (X), d'un angle (α42) compris entre 130 et 160°, ladite portion distale (42) ayant une dimension longitudinale comprise entre 15 et 30% de la dimension longitudinale totale de l'organe de rigidification dorsal (40), et s'étendant au moins partiellement dans la bande de jonction (13) adjacente, lesdites portions distales (42) étant configurées pour être placées de part et d'autre de la main, en position d'utilisation, sensiblement en regard des métacarpiens de l'index et de l'auriculaire.

7. Orthèse selon la revendication 5 ou 6, **caractérisée en ce que** chaque organe de rigidification dorsal (40) comporte une portion proximale (43) qui s'étend depuis l'extrémité proximale de la portion centrale (41) en étant inclinée dans la direction proximale et à l'opposé du bord longitudinal (4) voisin, par rapport à la direction longitudinale (X), d'un angle (α43) compris entre 145 et 175°, ladite portion proximale (43) ayant une dimension longitudinale comprise entre 15 et 30% de la dimension longitudinale totale de l'organe de rigidification dorsal (40).

8. Orthèse selon l'une des revendications 1 à 7, **caractérisée en ce qu'**elle comprend un organe de rigidification antérieur (45) qui comporte une portion distale (46) bombée dans la direction postérieure, destinée à être reçue dans la paume de la main, prolongée par une plaque (47) allongée s'étendant sensiblement jusqu'au bord proximal (3) de la nappe (1).

9. Orthèse selon l'une des revendications 1 à 8, **caractérisée en ce qu'**elle comprend un dispositif de serrage (50) comportant :
- une pluralité d'œillets (51) agencés dans la nappe (1) le long de chacun des bords longitudinaux (4) ;
- une patte de traction (52) comportant deux œillets (53) et possédant des moyens d'accrochage amovible (52a) sur l'enveloppe (10) ;
- et un lacet (54) formant une boucle fermée, le lacet (54) possédant une partie de laçage (55) passant successivement dans les œillets (51) de la nappe (1), et une partie de serrage (56) comportant un premier brin (57) et un deuxième brin (58) s'étendant vers l'extérieur de l'enveloppe (10) respectivement depuis un premier œillet (51a) et un deuxième œillet (51b) de la nappe (1), les parties extrêmes des deux brins (57, 58) passant chacune dans un œillet (53) de la patte de traction (52) et étant reliées par un brin de liaison (59) ;
le dispositif de serrage (50) étant configuré de sorte qu'une traction exercée sur la patte de traction (52), sensiblement orthogonalement à direction longitudinale, conduise au rapprochement des bords longitudinaux (4) de l'enveloppe (10) grâce au lacet (54), le dispositif de serrage (50) étant alors à l'état serré, le lacet (54) possédant une longueur suffisante pour que, à l'état serré, la partie de serrage (56) puisse être enroulée autour de l'enveloppe (10) sur au moins un tour, l'accrochage amovible de la patte de traction (52) sur l'enveloppe (10) permettant de maintenir le dispositif de serrage (50) à l'état serré.

10. Orthèse selon la revendication 9, **caractérisée en ce que** le premier œillet (51a) est situé au voisinage de l'un des bords longitudinaux (4) de l'enveloppe (10) et du bord proximal (3) de l'enveloppe (10), et **en ce que** le deuxième œillet (51b) est situé au voisinage du même bord longitudinal (4) et de préférence adjacent au premier œillet (51a), dans la direction distale.

11. Orthèse selon l'une des revendications 1 à 10, **caractérisée en ce que** les bords longitudinaux (4) de la nappe (1) sont incurvés, lorsque la nappe (1) est dans un état plan.

12. Orthèse selon l'une des revendications 1 à 11, **caractérisée en ce que**, lorsque la nappe (1) est dans un état plan, elle présente une ligne longitudinale médiane (A1) de symétrie, la nappe (1) présentant deux orifices (5) de passage du pouce, de sorte que l'orthèse (100) est ambidextre.

13. Orthèse selon l'une des revendications 1 à 12, **caractérisée en ce qu'**elle comprend un pan de textile (60) assemblé entre les bandes d'extrémité (12) de la nappe (1), lorsque la nappe (1) est enroulée sur elle-même, de sorte que l'orthèse (100) forme un manchon circonférentiellement fermé dans lequel un utilisateur peut insérer sa main sensiblement longitudinalement.
